# EUROPEAN PATENT APPLICATION

(11) **EP 1 920 765 A1**
(43) Date of publication of application: **14.05.2008**
(21) Application number: 06023155.2
(22) Date of filing: 07.11.2006
(51) Int. Cl.: A61K 9/127, B01D 1/18

(54) **Liposome preparation by single-pass process**

(71) Applicant: MediGene AG, 82152 Planegg/Martinsried (DE); Ludwig-Maximilians-Universität München, 80359 München (DE)
(72) Inventor: The designation of the inventor has not yet been filed
(74) Representative: Weiss, Wolfgang

(57) **Abstract**

The present invention relates to a method of preparing liposomes in a single-pass mode. The method comprises the extusion of a solution or suspension through a porous device and subsequently passing said suspension or solution through a nozzle. Passing the suspension through said nozzle may result in an atomisation of the suspension or solution into droplets which might be employed in a subsequent spray-drying or spray-freezing process.

## Description

### Introduction

The present invention relates to a method for the preparation of liposomes in a single-pass mode. The method comprises the extrusion of a solution or suspension comprising lipids through a porous device an subsequently passing said solution or suspension through a nozzle.

Liposomes are lipid vesicles of closed bilayers entrapping an aqueous volume. Liposomes may be unilamellar vesicles, constituted by a single bilayers membrane, or multilamellar vesicles (MLV), constituted by multiple concentric bilayers. Depending on their size, unilamellar vesicles are roughly classified into small unilamellar liposomes (SUV) with an particle size between about 25 - 100 nm and large unilamellar liposomes (LUV) with an particle size between about 100 nm - 10 µm. For the pharmaceutical use unilamellar liposomes are preferred over multilamellar liposomes.

In recent years liposomes have become an important tool in the pharmaceutical industry for the delivery of drugs. Hydrophobic drugs are formulated in liposomes by the integration of the drug into the lipid bilayers. Hydrophilic agent may be formulated in liposomes by encapsulation in the aqueous core of the liposomes. Liposomes are capable of influencing pharmacokinetics by a sustained release of the drug to the body or reduce side effects by limiting the free concentration of a drug. By attaching ligands to the liposome or rendering their charge, liposomes facilitate a targeted delivery of dugs to a desired site of action. Beside the pharmaceutical use, liposomes are also frequently used for cosmetic products.

For the pharmaceutical administration of liposomal the particle size is a parameter of major concern. The optimal size range for parenteral administration is between about 70 and 400 nm. Within this size range liposomes favour biodistribution into certain target organs such as liver, spleen and bone marrow. Furthermore liposomes within this size-range have a good stability in blood and display predictable dug-release rates. Liposomes of greater than 400 nm have a greater tendency to aggregate. At a size of about 5 µm, liposomes injected into the circulation may block capillaries. Thus, size distribution is an important topic in the application of liposomes.

A variety of principal procedures have been developed for the initial dispersion of lipids in an aqueous system for the preparation of liposomal suspensions. Commonly used methods include the hydratisation of a dry lipid film with a desired aqueous medium ("film method", "hand shaken method") or the dispersion of an organic solvent solution of lipids into an aqueous medium ("ethanol/ether-injection"). The resulting liposomal suspensions usually have a broad size distribution and heterogeneous lamellarity. Predominantly MLV can be observed as the initial product. Different size processing methods have been developed to produce liposomes of desired size range and uniformity.

Liposomes may be sized by sonication of larger and multilamellar liposomes. Resulting liposomes are SUV in the size range of about 25 - 80 nm. However, a narrow size distribution can only be achieved at liposome sizes of about less than 50 nm. Since SUV have only a limited drug loading capacity and unfavourable pharmacokinetic and pharmakodynamic properties, sonication is not a suitable method for liposomes destined for a pharmaceutical applications. Other drawbacks of this method are the limited processing volume, long sonication times and possible oxidation of the lipids due to heat stress. Therefore, the method is not well suitable for regular industrial manufacturing.

Alternatively, large multilamellar liposomes may be sized and homogenized by a high pressure approach. In a discontinuous process, a liposomal suspension is extruded at high pressure (up to about 160 MPa) through a small orifice, for example using a "French Press"-apparatus as described by Hunt and Papahadyopoulous (US 4,529,561) or by Brandl et al (WO 96/05808). This process is repeated until the desired size distribution is achieved. Homogenisation chambers or homogenisation nozzles have been especially designed for the use with high pressure pumps as disclosed for example in DE 3905354 or US 6,331,314. The "high pressure homogenisation" is also suitable for the preparation of liposomes from lipid/water dispersions without the use of organic solvent (Brandl, Martin M. et al, Liposome preparation using high-pressure homogenizers. Liposome Technol. (2nd Ed.) (1993), 1, 49-65). Typically SUVs with quite broad and variable distribution are produced. The use of high pressure pumps has enabled a cycling through an orifice at high pressure, rendering possible the processing of larger batch sizes. Still, the process is carried out in a discontinuous manner.

For the production of LUV with a defined size between about 100 - 400 nm and a narrow size distribution, methods of size-processing based on the extrusion of liposomal suspension through a uniform pore size membrane have been developed (Szoka et al 1979, Olsen et al 1979). Preferably polycarbonate membranes, usually of a pore size between about 50 - 200 nm are employed in this method. The selection of the membrane pore size allows a predetermination of the desired liposome size. To obtain liposomes of high homogeneity, liposomes can be extruded through the membrane multiple times (Cullis WO 86/00238). For the processing of larger batch sizes, liposomal preparation can be extruded through the membrane in a continuous recycling by a pump until the desired size and polydispersity has been achieved (Janoff EP 0460720). In general the polydispersity of the liposomes is deceased with increasing numbers of extrusion cycles.

To obtain a narrow, well defined, size distribution of the product and reduce the number of extrusion steps required, several variations of the extrusion membrane are known in the prior art. By applying a polymer membrane having a web-like structure for the extrusion process, Morano et al US 4,927,637 achieves good polydispersity. Martin et al US 4,737,232 discloses an asymmetric membrane, whereas Suddith (US 5,556,580) uses a frit instead of a membrane for liposomal extrusion.

Different approaches to optimise the method employ high pressure for the extrusion process. For example Sachse et al (DE 4328331) use pressures of 6,5 MPa for the extrusion through a series of staggered membranes. Hill at (US 2005/0260256) enables the use of up to about 55 MPa for the extrusion by using hydrophilic screen membranes in a support cassette holder.

For the large scale production of liposomes with a suitable size and narrow size distribution which has to be met for the pharmaceutical application of drug loaded liposomes, usually repeated processing of the liposomal material either by the high pressure homogenisation or the membrane extrusion method is still required. Hence, a single pass method for the continuous preparation of appropriately sized liposomes can not be performed with these standard methods.

Aqueous liposomal preparations are usually not stable over a long period of time which is required for the industrial manufacture and supply process. A standard method for preserving liposomes is the dehydration of the aqueous liposomal preparations. Dehydration may be achieved by spray-drying or spray-freeze-drying which both requires the atomisation of the suspension.

It was the underlying problem of the current invention to provide a continuous method for the preparation of liposomal suspensions with a suitable size and size distribution for a parenteral application. It was another problem to provide a continuous method for the preparation of such liposomal suspensions and the atomisation of the suspensions for the subsequent drying procedure. Furthermore, scalability of the process and the use of established standard material was desired.

### Summary of the invention

The current invention provides a continuous method for the preparation of liposomes from a suspension or solution. The starting suspension or solution may comprise lipids which may preferably be in the form of lipid particles. The lipid particles in the starting suspension may be heterogeneously sized, whereas the liposomes obtained by the method may be characterized by a homogeneous size. The method comprises extruding the suspension or solution first through a porous device and subsequently passing the suspension or solution through a nozzle in a continuous mode. The method is performed in a single-pass mode, wherein the suspension or solution is extruded through the porous device and through the nozzle only once. Preferably the liposomes obtained by the method comprise are homogeneously sized. By the inventive process, the average particle size distribution of lipid particles of the starting suspension is reduced to a smaller particle size. The method may also include the provision of the solution or suspension comprising lipids and the collection of the prepared liposomes.

Thus, the invention relates to method for the preparation of liposomes in a single-pass mode, comprising the steps:
(a) providing a suspension or solution comprising lipids,
(b) extruding the suspension or solution of step (a) through a porous device, and subsequently
(c) passing said suspension or solution of step (b) through a nozzle, and
(d) optionally collecting the liposomes formed after step (c).

The method may be performed at high pressure of up to about 1200 bar, which is generated by suitable pumps. By restricting the flow of the suspension or solution by means of a suitable nozzle, the pressure of the suspension or solution is about equal on both sides of the porous device. Preferably the porous device, through which the liposomes are extruded, has a pore size of 50-300 nm, more preferably of 100-200 nm.

Although homogenisation of liposomes by extrusion through membranes or high pressure extrusion through nozzles or orifices was known before, it could not be predicted, that the combination of the two methods provides a continuous process suitable for a the preparation of liposomes with a narrow size distribution and an average size between 50 nm and 500 nm, preferably from about 50 nm to about 200 nm. The latter product characteristics are preferred for the use of liposomes in a parenteral medical application. To obtain such characteristics by established extrusion techniques, several extrusion steps have to be performed which prevents a continuous set up of the process.

The extrusion of liposomes through a porous device with a predetermined pore size is known as a restrictive method for the sizing of liposomes. Therefore it was even more unexpected that the size distribution of liposomes can be improved by subsequently passing the liposomes through a nozzle. The size distribution of the liposomes is characterized by the polydispersity index. Thus, a decrease of the polydispersity index indicates a decrease of the breadth of the size distribution.

Given that the suspension or solution comprising lipids is maintained under high pressure after the porous device, passing said suspension or solution through the nozzle enables a spraying/atomisation. The atomisation may be performed in a device that is suitable for either drying or freezing the atomised suspension in a fashion commonly performed in a spray-drying or spray-freezing process. Preferably, the drying/freezing may be carried out by percolative drying as disclosed in co-owned European application "Percolative drug for the preparation of particles" filed on 27.10.2006.

It is a further aspect of the current invention to provide a continuous method for the extrusion and spray-drying or spray-freezing of liposomes, comprising the extrusion of a starting suspension or solution comprising lipids through a porous device and subsequently passing the suspension or solution through a nozzle, thereby atomising the suspension or solution.

The invention furthermore discloses an apparatus comprising a porous device, which is connected to nozzle via a conduit. The apparatus is suitable for performing the method as disclosed by the invention.

### Definitions

"About" in the context of amount values refers to an average deviation of maximum +/-20 %, preferably +/-10 % based on the indicated value. For example, an amount of about 30 mol% cationic lipid refers to 30 mol% +/-6 mol% and preferably 30 mol% +/-3 mol% cationic lipid with respect to the total lipid/amphiphile molarity.

"Active compound" refers to a compound, or mixture of compounds, which has a particular bioactivity based on which it is useful as an agent useful for the diagnosis, prevention, or treatment of a human or animal disease or condition. Drug substances, diagnostic compounds and vaccines are important examples of active compounds according to the present invention.

"Aqueous medium" or "aqueous liquid" is a liquid material which contains water. The material may represent a single liquid phase, or a two- or multiphase system, wherein the continuous phase is liquid and contains water. An aqueous suspension, a dispersion or an emulsion, wherein the continuous phase is aqueous are also examples of aqueous medium. An aqueous medium which contains a colloidal material is hereinafter sometimes referred to as an aqueous colloidal dispersion or solution.

"Cryogenic fluids" are materials that are liquid in the temperature range which is necessary to freeze aqueous solutions, preferably they are applied at temperatures below -90°C. The cryogenic fluid can freeze particles and/or pellets in-situ by getting in contact with the cryogenic fluid.

"Drain disk" is a device with a filter like structure with a pare pore size.

"Dry" or "dehydrate" are used interchangeable and relate to the process of removing an aqueous medium by evaporation or sublimation.

"Formation of droplets" refers to the conversion of a material into fine particles or pellets by measures like pressure atomisation, two fluid atomisation, centrifugal droplets formation and nozzles like e.g. piezo droplet forming devices, sound activated-, magnetic- and electrostatic droplet forming devices.

"Hydrophilic excipient" refers to a pharmaceutically acceptable, pharmacologically substantially inert material with hydrophilic properties. Hydrophilicity means that the hydrophilic excipient should be substantially water soluble.

"Homogeneity" or "homogeneous" as used herein refers to a narrow size distribution of a particle population. The high size homogeneity or narrow size distribution is characterized by a low polydispersity index.

"Lipid" refers to its conventional sense as a generic term encompassing fats, lipids, alcohol-ethersoluble constituents of protoplasm, which are insoluble in water. Lipids are composed of fats, fatty oils, essential oils, waxes, steroid, sterols, phospholipids, glycolipids; sulpholipids, aminolipids, chromolipids, and fatty acids. The term encompasses both naturally occurring and synthetic lipids. Preferred lipids in connection with the present invention are: steroids and sterol, particularly cholesterol, phospholipids, including phosphatidyl and phosphatidylcholines and phosphatidylethanolamines, and sphingomyelins. Where there are fatty acids, they could be about 12-24 carbon chains in length, containing up to 6 double bonds. The fatty acids are linked to the backbone, which may be derived from glycerol. The fatty acids within one lipid can be different (asymmetric), or there may be only 1 fatty acid chain present, e. g., lysolecithins. Mixed formulations are also possible, particularly when the non-cationic lipids are derived from natural sources, such as lecithins (phosphatidylcholines) purified from egg yolk, bovine heart, brain, or liver, or soybean.

"Liposomes" are artificial lipid bilayer vesicles of various sizes and structures. Unilamellar vesicles are liposomes defined by a single lipid bilayer enclosing an aqueous space. In contrast, oligo- or multilamellar vesicles comprise several membranes. Typically, the membranes are roughly 4 nm thick and are composed of amphiphilic lipids, such as phospholipids of natural or synthetic origin. Optionally, the membrane properties can be modified by the incorporation of other lipids such as sterols or cholic acid derivatives. Liposomes with particularly flexible membranes based on phospholipids with a low phase transition temperature (i.e. below body temperature) are sometimes referred to as transfersomes.

Depending on their diameter and number of bilayer membranes, liposomes may also be classified as multilamellar vesicles (MLV, two or more bilayers, typically above approx. 150 to 200 nm), small unilamellar vesicles (SUV, one single bilayer, typically below about 100 nm), multivesicular vesicles (MVV, several vesicular structures within a larger vesicle), and large unilamellar vesicles (LUV, one single bilayer, typically larger than about 100 nm).

A "nozzle" as used herein refers to a conduit with a variable cross-sectional area in which a fluid accelerates into a high-velocity stream. The fluid must be compressed to a state of high pressure before it is sent through the nozzle.

"Paclitaxel" (which should be understood herein to include analogues, formulations, and derivatives such as, for example, deacetylpaclitaxel, deacetyl-7-epipaclitaxel, docetaxel, taxotere (a formulation of docetaxel), 10-desacetyl analogs of paclitaxel and 3'N-desbenzoyl-3'N-t-butoxycarbonyl analogs of paclitaxel) may be readily prepared utilizing techniques known to those skilled in the art (see also WO 94/07882, WO 94/07881, WO 94/07880, WO 94/07876, WO 93/23555, WO 93/10076; U. S. Pat. Nos. 5,294, 637; 5,283, 253; 5,279, 949; 5,274, 137; 5,202, 448; 5,200, 534; 5,229, 529; and EP 590,267), or obtained from a variety of commercial sources, including for example, Sigma Chemical Co., St. Louis, Mo. (T7402 from Taxus brevifolia; or T-1912 from Taxus yannanensis). Paclitaxel should be understood to refer to not only the common chemically available form of paclitaxel, but analogs (e. g., taxotere, as noted above) and paclitaxel conjugates (e.g., paclitaxel-PEG, paclitaxeldextran, or paclitaxel-xylose).

The term "taxane" as used herein refers to the class of antineoplastic agents having a mechanism of microtubule action and having a structure that includes the unusual taxane ring structure and a stereospecific side chain that is required for cytostatic activity. Also included within the term "taxane" are a variety of known derivatives, including both hydrophilic derivatives, and hydrophobic derivatives. Taxane derivatives include, but not limited to, galactose and mannose derivatives described in International Patent Application No. WO99/18113; piperazino and other derivatives described in WO 99/14209; taxane derivatives described in W099/09021, WO 98/22451, and U. S. Patent No. 5,869, 680; 6-thio derivatives described in WO 98/28288; sulfenamide derivatives described in U. S. Patent No. 5,821, 263; and taxol derivative described in U. S. Patent No. 5,415, 869.

As used herein, "thermally sensitive", "temperature-sensitive" or "thermally labile" means that a material is incompatible with drying methods based on the evaporation of water by heat, such as spray drying. In other words, a thermally labile material looses at least some of its structure, activity, functionality or chemical purity when treated by such thermal drying methods, so that the product may be pharmaceutically unacceptable.

"Pharmaceutically acceptable" is meant to encompass any substance, which does not interfere with effectiveness of the biological activity of the active ingredient and that is not toxic to the host to which it is administered.

"Polydispersity" is the width of the distribution of the particle size in a given particle sample. The Polydispersity is characterised by the polydispersity index.

"Polydispersity index" (PDI) is an estimation of the wide of the distribution of the particle size in a given liposomal sample. Before calculation of the PDI the particle size will be determined by dynamic light scattering (DLS). This technique measures the time-dependent fluctuations in the intensity of scattered light which occur because the particles are undergoing Brownian motion. Analysis of these intensity fluctuations enables the determination of the diffusion coefficients of the particles which are converted into a size distribution. The calculations of these parameters are defined in the ISO standard document 13321:1996 E.

"Single-pass mode" or "single-pass process" refers to a method wherein the processed material passes a processing step only one time. Thus, the suspension, solution or liposomes employed in the inventive method pass the porous device or the nozzle employed in the current invention only once.

Lipid particles used in the current invention may be liposomes, lipid complexes, solid lipid nanoparticles, lipoplexes, niosomes, micelles or mixed micelles, oligo- or multilamellar vesicles. Preferably, the lipid particles are liposomes. It is the most preferred embodiment of the invention that the liposomes are cationic liposomes. The method might also employ particles formed through the association of amphiphilic molecules such as detergents. In contrast to liposomes, these structures are based on monolayers which are less stable and tend to disassemble upon dilution. Micelles are e.g. disclosed in WO 02/085337 and EP-A 730 860, the teachings of which are incorporated herein by reference.

The lipids or lipid particles are provided in a suspension or solution comprising an aqueous medium. Besides water, the aqueous medium of the

present invention may comprise one or more further liquid constituents which are at least partially miscible with water, preferably organic solvents, more preferably alcohols (e.g. C₁₋₄ alcohols such as methanol, ethanol, propanol, butanol and combinations thereof, etc.) or ketones (e.g. C₁₋₄ ketones such as acetone, methylethyl-ketone, DMF, DMSO and combinations thereof, etc.). In a preferred embodiment of the invention, the aqueous medium is water or a mixture of water and ethanol. The ratio between water and the further liquid constituent is preferably between about 99.9:0.1 and about 10:90 (v/v), more preferably between about 70:30 and about 40:60 (v/v), most preferably between about 80:20 and about 60:40 (v/v).

The use of said organic solvents, preferably ethanol in the ratio described, in the combination with the further embodiments of the invention provides a variety of advantages over previously known systems for producing unilamellar liposomes, including the following:
1) the method results in very small and homogenous liposomes;
2) the ability to use high lipid concentrations (e.g., on the order of 100 mM) so as to easily achieve high loading efficiencies;
3) a decreased viscosity, which leads to an increased fluidity, enabling high flow rates of the lipid suspension or solution;
4) the use of organic solvents leads to a reduction in drying temperature during the evaporation process.

The liposomes prepared by the inventive method may have different sizes, lamellarity and structure. Preferably the liposomes prepared by the method of the invention have an average diameter of about 50 nm to about 500 nm. Most preferred is a size of about 50 to about 200 nm. Preferably the liposomes are unilamellar liposomes.

The suspension or solution, lipid particles or liposomes used within the context of the present invention may comprise neutral, anionic and/or preferably cationic lipids. Cationic lipids are preferably comprised in an amount of at least about 30 mol%, more preferably of at least 40 mol% and most preferably in an amount of at least 50 mol% of total liposome forming lipids. Neutral or anionic lipids may be selected from sterols or lipids such as cholesterol, phospholipids, lysolipids, lysophospholipids, sphingolipids or pegylated lipids with a neutral or negative net charge. Useful neutral and anionic lipids thereby include: phosphatidylserine, phosphatidylglycerol, phosphatidylinositol (not limited to a specific sugar), fatty acids, sterols, containing a carboxylic acid group for example, cholesterol, 1,2-diacyl-sn-glycero-3-phosphoethanolamines, including, but not limited to, 1,2-dioleylphosphoethanolamine (DOPE), 1,2-dihexadecylphosphoethanolamine (DHPE), 1,2-diacyl-glycero-3-phosphocholines, including, but not limited to 1,2-distearylphosphatidylcholine (DSPC), 1,2-dipalmitylphosphatidylcholine (DPPC), 1,2-dimyristylphosphosphatidylcholine (DMPC), phosphatidylcholine preferably egg PC, soy PC and sphingomyelin. The fatty acids linked to the glycerol backbone are not limited to a specific length or number of double bonds. Phospholipids may also have two different fatty acids. Preferably the further lipids are in the liquid crystalline state at room temperature and they are miscible (i.e. a uniform phase can be formed and no phase separation or domain formation occurs) with the used cationic lipid, in the ratio as they are applied. In a preferred embodiment the neutral lipid is 1,2-dioleylphosphatidylcholine (DOPC). Further, the neutral and/or anionic lipids may comprise poly(ethylene) glycol chains.

Cationic lipids may preferably be selected from a group comprising N-[1-(2,3-dioleoyloxy)propyl]-N,N,N-trimethyl ammonium (TAP) salts, preferably the chloride or methylsulfate. Preferred representatives of the family of TAP lipids are DOTAP (dioleoyl-), DMTAP (dimyristoyl-), DPTAP (dipalmitoyl-), or DSTAP (distearoyl-). Other useful lipids for the present invention may include, but are not limited to: DDAB, dimethyldioctadecyl ammonium bromide; 1,2-diacyloxy-3-trimethylammonium propanes, (including but not limited to: dioleoyl, dimyristoyl, dilauroyl, dipalmitoyl and distearoyl; also two different acyl chains can be linked to the glycerol backbone); N-[1-(2,3-dioloyloxy)propyl]-N,N-dimethyl amine (DODAP); 1,2-diacyloxy-3-dimethylammonium propanes, (including but not limited to: dioleoyl, dimyristoyl, dilauroyl, dipalmitoyl and distearoyl; also two different acyl chain can be linked to the glycerol backbone); N-[1-(2,3-dioleyloxy)propyl]-N,N,N-trimethylammonium chloride (DOTMA); 1,2-dialkyloxy-3-dimethylammonium propanes, (including but not limited to: dioleyl, dimyristyl, dilauryl, dipalmityl and distearyl; also two different alkyl chain can be linked to the glycerol backbone); dioctadecylamidoglycylspermine (DOGS); 3β-[N-(N',N'-dimethylamino-ethane)carbamoyl]cholesterol (DC-Chol); 2,3-dioleoyloxy-N-(2-(sperminecarboxamido)-ethyl)-N,N-dimethyl-1-propanaminium trifluoro-acetate (DOSPA); β-alanyl cholesterol; cetyl trimethyl ammonium bromide (CTAB); diC14-amidine; N*-tert*-butyl-N'-tetradecyl-3-tetradecylamino-propionamidine; 14Dea2; N-(alpha-trimethylammonioacetyl)didodecyl-D-glutamate chloride (TMAG); O,O'-ditetradecanoyl-N-(trimethylammonio-acetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butane-diammonium iodide; 1-[2-(acyloxy)ethyl]2-alkyl(alkenyl)-3-(2-hydroxyethyl)-imidazolinium chloride derivatives as described by Solodin et al. (Solodin et al., 1995), such as 1-[2-(9(Z)-octadecenoyloxy)ethyl]-2-(8(Z)-heptadecenyl-3-(2-hydroxyethyl)imidazolinium chloride (DOTIM), 1-[2-(hexadecanoyloxy) ethyl]-2-pentadecyl-3-(2-hydroxyethyl)imidazolinium chloride (DPTIM), 2,3-dialkyloxypropyl quaternary ammonium compound derivatives, containing a hydroxyalkyl moiety on the quaternary amine, as described e.g. by Felgner et al. (Felgner et al., 1994) such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropyl ammonium bromide (DORIE-HP), 1,2-dioleyl-oxy-propyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), 1,2-disteryloxypropyl-3-dimethyl-hydroxyethyl ammonium bromide (DSRIE); cationic esters of acyl carnitines as reported by Santaniello et al. (US 5,498,633); cationic triesters of phospahtidylcholine, i.e. 1,2-diacyl-sn-glycerol-3-ethylphosphocholines, where the hydrocarbon chains can be saturated or unsaturated and branched or non-branched with a chain length from C₁₂ to C₂₄. Preferably, the cationic lipids comprise two acyl chains, which may be different or identical.

It is an aspect of the present invention that the starting suspension or solution comprising lipids, preferably lipid particles, and the liposomes obtained by the inventive method may comprise an active compound. Preferably, the active compound is a pharmaceutically active compound. The active compound may be incorporated within or associated with the liposomes and/or lipid particles. In the case of liposomes, for example, a hydrophilic active compound may be encapsulated within the aqueous inner space of the liposomes, whereas a lipophilic active compound is usually associated with the membrane-forming lipids. In the case of lipid nanoparticles, lipophilic compounds are also easily incorporated within the matrix of such nanoparticles.

Liposomes and/or lipid particles are particularly suitable drug carriers for active compounds, in particular pharmaceutically active compounds, which are not easy to deliver effectively, such as very poorly soluble compounds, sensitive (including thermally labile) active compounds, peptides, proteins, and nucleic acids including DNA, RNA, iRNA, siRNA, or oligonucleotides. Another preferred group of active compounds are those which are more effectively delivered in colloidal form because the distribution of colloids in the organism after intravenous injection is more favorable with regard to efficacy or side effects than the administration of a solution of the compound. Preferred examples of pharmaceutically active compounds include, but are not limited to, antimicrobial, antifungal, antiviral, and cytostatic or cytotoxic agents. Examples of such agents include doxorubicin, mitoxanthrone, and amphotericin B.

Liposomes used within the context of the invention are especially suitable for the encapsulation of hydrophobic drugs. Examples are taxanes, camptothecins, doxorubicin, michellamine B, vincristine, and platinum compounds such as cisplatin. Preferred examples of such hydrophobic drugs are paclitaxel, docetaxel and camptothecins, which have a low solubility in water and/or are heat sensitive.

In a specially preferred embodiment of the invention, the liposomes obtained by the method comprise DOTAP used in the form of DOTAP chloride, DOPC and paclitaxel, preferably in a ratio of about 50:47:3. This formulation is also designated MBT-0206 or EndoTAG-1. EndoTAG-1 has a lipid content of about 10 mM in a about 10% m/m trehalose dihydrate solution. The preparation of such a liposomal preparation is disclosed in WO 2004/002468.

The liposomes obtained according to the present invention can be administered orally, transdermally, intravenously, intrabrochially, intramuscularly, intraoculary, subcutaneously and interperitoneally. As a drug delivery system, liposomes can significantly change the pharmacokinetic and pharmacodynamic fate of a compound by enhancing drug uptake, delaying the loss of rapidly cleared drugs and reducing drug toxicity. They have widely been investigated for the delivery of chemotherapeutic agents for treatment of cancer, antimicrobial agents for treatments of bacterial, viral and parasitic diseases, and also for use as immunological adjuvans for delivery of vaccines.

Liposomes may be labile in various respects. Their physical structure may be sensitive to heat, so that a highly thermal drying method would lead to the disassembly of the liposomal membranes, to the loss of functionality or to the loss of incorporated or associated active compound. Depending on their composition, they may also be chemically labile to heat. For example, some lipids hydrolyse or oxidise rapidly in an aqueous environment at elevated temperatures.

In a preferred embodiment, the starting suspension or solution comprising lipids or lipid particles optionally comprises a hydrophilic excipient. Useful hydrophilic excipients can be monomeric, oligomeric or polymeric and may be found among several chemical classes of compounds. According to one of the preferred embodiments of the invention, the hydrophilic excipient is a saccharide, e.g. a mono-, di-, oligo- or polysaccharides, a sugar alcohol, an amino acid, a peptide, a protein, a water-soluble polymer or a combination thereof.

A saccharide, or carbohydrate, is defined as a compound predominantly composed of carbon, hydrogen, and oxygen. Useful saccharides include sugar and sugar alcohols, oligosaccharides, water soluble polysaccharides and derivatives thereof. Preferred saccharides according to the invention include, but are not limited to, glucose, fructose, lactose, sucrose, trehalose, maltose, cellobiose, galactose, maltotriose, maltopentose, raffinose, dextrin, dextran, inulin, mannitol, sorbitol, xylitol, chitosan; water soluble cellulose derivatives such as methylcellulose, hydroxypropylcellulose, hydroxyethylcellulose, and hypromellose; alginates, soluble starches or starch fractions, xanthan gum, guar gum, pectin, carrageen, galactomannan, gellan gum, tragacanth, including any derivatives of these. Particularly preferred saccharides are glucose and trehalose.

Other useful hydrophilic excipients may be selected from other chemical classes, such as from water soluble amino acids, peptides or proteins. For example, glycine or other natural amino acids may be used. Useful proteins include, but are not limited to, gelatine, albumin, whey protein, soy protein, or other food or vegetable proteins.

Still further examples of useful hydrophilic excipients are polymers such as water soluble polymers such as solid polyethylene glycols, polyvinylalcohol, polyacrylates, or polyvinylpyrrolidone.

According to the invention, mixtures of more than one hydrophilic excipient may be used. For example, there may be a need to adjust several parameters such as pH, solubility, and wettability independently. In this case, a first hydrophilic excipient may be selected as a basic carrier material for the colloidal systems, whereas one or more further hydrophilic excipients may be incorporated to obtain a certain pH and/or wettability.

The content of the hydrophilic excipient, or mixture of hydrophilic excipients, in the aqueous phase may vary widely, depending on its aqueous solubility and other considerations, and be even up to about 80 wt.-%. More preferably, it is from about 0.1 to about 65 wt.-%. A content of about 3 to about 60 wt.-%, and particularly from about 5 to about 50 wt.-% is preferred.

Of course, the aqueous medium may comprise further excipients or auxiliary substances, which may or may not be hydrophilic or water soluble. Depending on their nature and that of the extraction medium, i.e. on whether or not these substances are soluble in and extracted by the extraction medium, such substances can be comprised into the dry particles or removed together with the water and organic solvent. Substances, which are comprised into the dry particles, should be pharmaceutically acceptable.

Further excipients which may be useful in particles formulations, and in particular in particles formulations which are used for reconstitution and optionally, parenteral administration, are generally known to pharmaceutical formulation scientists. Preferred further excipients include stabilisers, surfactants, wetting agents, bulking agents, lyophilisation aids, antioxidants, chelating agents, preservatives, osmotic agents, acidic or alkaline excipients for adjusting the pH, etc.

Among the preferred excipients according to the invention are stabilisers and antioxidants. Antioxidants may prevent the oxidation of an incorporated active compound, but also that of components of the colloidal in particular if lipids are used which are sensitive to oxidisation. Useful compounds include, for example, lipid-soluble antioxidants such as alpha-, beta-, and gamma-tocopherol, ubiquinol, lycopene, alpha- and beta-carotene, nordihydroguaiaretic acid, butyl hydroxyanisole, butyl hydroxytoluene, ethylenediamine tetraacetic acid, diethylenetriamine pentaacetic acid, desferal, p-hydroxybenzoic, vanillic, syringic, 3,4-dihydroxybenzoic, p-coumaric, ferulic, sinapic and caffeic acids, ascorbyl palmitate, carnosol, esculetin, esculin, fraxetin, fraxin, quercetin, morin, etc. Particularly preferred are alpha-tocopherol and ethylenediamine tetraacetic acid, including the pharmaceutically acceptable derivatives thereof. On the other hand, if chemically pure, semisynthetic or synthetic saturated lipids are used for composing the colloidal systems, no antioxidant may be needed.

As described before, it is an aspect of the current invention, that a starting suspension or solution comprising lipids and/or lipid particles is extruded through a porous device and subsequently passed through a nozzle in a continuous mode. The utilization of a nozzle with a sufficiently small diameter restricts the flow of the suspension after being extruded through the porous device and thereby pressurized the suspension. The sequential use of a porous device and a nozzle enables an about equally high pressure on both sides of the porous device when the inventive method is performed.

It is also an aspect of the invention to disclose an apparatus which is suitable to carry out the inventive method. The apparatus comprises at least one porous device which is connected to at least one nozzle and at least one pump, suitable for passing an suspension or solution comprising lipids through the porous device and the nozzle.

Thus, it is a further aspect of the invention to provide an apparatus for the preparation of liposomes comprising:
(i) a container for supplying a solution or suspension comprising lipids,
(ii) a porous device,
(iii) a nozzle,
(iv) means for connecting the container (i) with the porous device (ii) by a conduit,
(v) means for generating pressure, and
(vi) optionally means for dehydrating the solution or suspension comprising liposomes and
(vii) optionally means for collecting the obtained liposomes.

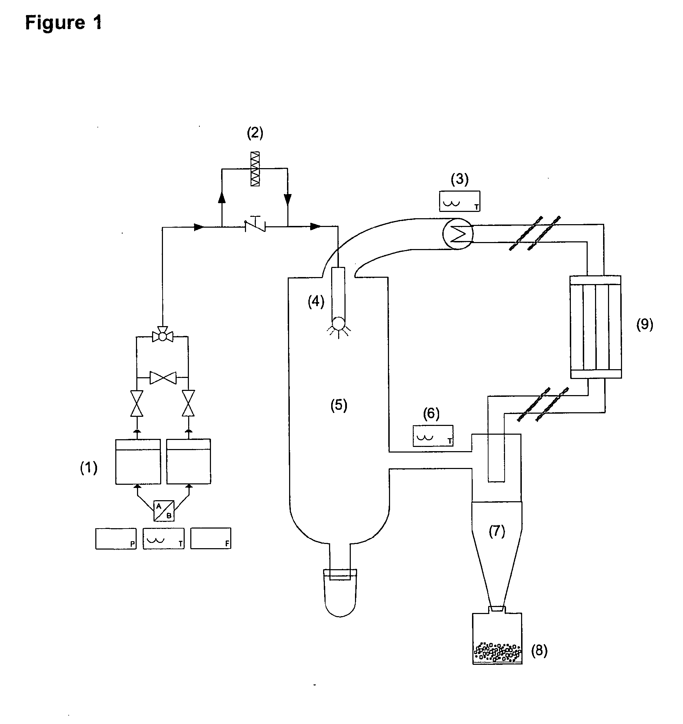

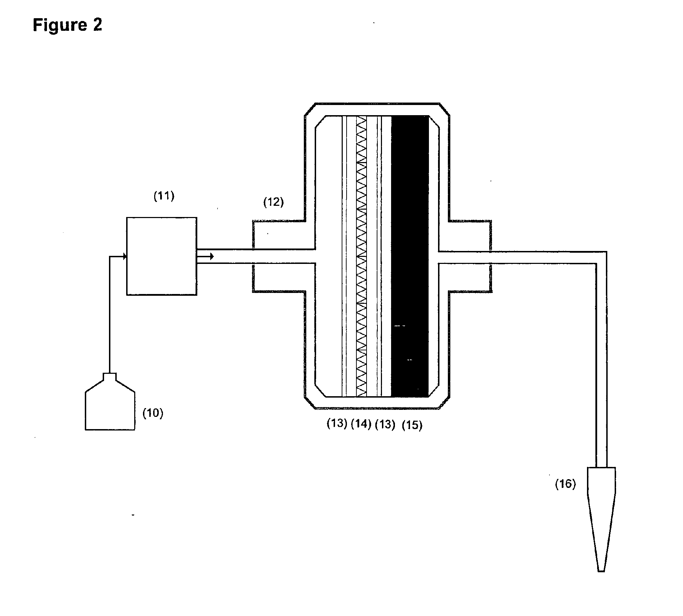

An illustration of preferred embodiments of the apparatus according to the present invention are shown in Figures 1 and 2.

The porous device used to prepare the liposomes by the inventive method or which is part of the inventive apparatus preferably has a pore size of between about 300 nm and about 50 nm, more preferably between about 200 nm and about 100 nm. The porous device comprises at least one porous layer, i.e. the porous device may comprise one porous layer or a plurality of identical or different porous layers. The porous device may be a membrane, a filter, a frit or any other restricting device comprising pores and combination thereof. The device may consist of a polymeric material, metal, glass, ceramic or any other suitable material. The material may be chemically inert, low absorbing for the constituents of the solution/suspension and wet able. In a preferred embodiment, the porous device is a membrane, preferably a polycarbonate membrane. In another embodiment the porous device may be a sterile filtration membrane. Polycarbonate membranes produced by Osmonics Inc., USA have been found to work successfully in the practice of the present invention. According to the present invention, the pressure on both sides of the porous device is preferably substantially identical.

Because the suspension is passed trough the porous device system only once, the porous device does not have to be changed due to pore clogging. Clogging in general depends on such variables as lipid composition, purity and concentration, as well as on the pressure and flow rates used. A second passage through a porous device is in no case objective of this invention.

The porous device, preferably a membrane, that is used for extrusion, may be supported by at least one drain disk on at least one side, preferably on both sides embedding the porous device. Drain disks of this type produced by Osmonics Inc., USA have been found to work successfully in the practice in the present invention.

The at least one drain disk may be further mechanically supported by at least one frit, especially on the porous device's side which is connected to the nozzle. Such types of membrane holders are generally known to the skilled worker in the art.

The frit element is a structural member comprised of generally bead-like material partially conjoined, usually by compression and/or sintering. Among the many materials useful in frit preparation are ceramic, glass metal and metal/carbide bead-like materials which are conjoined into a frit. Due to the low affinity of lipid for metal, metal frits are preferred for facilitating substantially total through-put of material. The preferred metal frit is of stainless steel. Paths through the frit are not straight paths and path walls are irregular. Frits may be prepared in a number of shapes including rod or disc shapes. Suitable frits are available from a number of sources such as Ace Scientific Company, (East Brunswick, N.J.), Scientific Systems, Inc. (State College, Penn.) and Ranin Instrument Company (Woburn, Mass.). Frits are characterized in having non-uniform pore size over a pore size range. Frit pore size range may be experimentally determined on the basis of material that passes through or is excluded by the frit. This defines a range of particle sizes a portion of which lie above and below, the stated pore size. Preferred pore sizes are ≥ 1 µm, pref. 1-50 µm, more preferably 5-20 µm.

The increased strength of the embedded porous device, preferably of the embedded membrane which is supported by drain disks and optionally at least one frit, enables high flow rate of extrusion through the membrane. The porous device, preferably the membrane does not suffer occlusion and is not deformed by the pressure of the extrusion step. Surprisingly membrane type extrusion is not rate limited by the minimal resistance to deformation of membrane material.

A very preferred embodiment of the present invention is shown in Figure 2, wherein the assembly of a preferred porous device is illustrated. In particular, this preferred porous device comprises a membrane, preferably a polycarbonate membrane (14) which is supported on both sides by drain disks (13). Further, the preferred porous chain comprises a further porous material, preferably a frit, which is located on the porous device's side which is connected with the nozzle.

It is a further aspect of the current invention, that after the extrusion through the porous device, the suspension or solution of lipids/liposomes is subsequently passed through a nozzle. Useful nozzles to carry out this step of the method of the invention or which are part of the inventive apparatus are also generally known to the skilled worker in the field. They include, for example, rotating disk nozzles, impinging jet nozzles, capillary nozzles, single orifice nozzles, ultrasonic nozzles of vibrating or pulsating type, two-fluid nozzles such as coaxial two-fluid nozzles etc. In a preferred embodiment of the invention the nozzle is an orifice nozzle. The nozzle used to further improve the liposomes or the unimodal distribution of liposomes was an orifice nozzle 121 VG ¼ produced by Schlick atomization technologies, Untersiemau, Germany. Within the current invention, the preferred pore size of the nozzle is between about 0.05 mm to about 1 mm, more preferably between about 0.1 mm to about 0.2 mm. In the inventive apparatus, the nozzle may be comprised in a container suitable for dehydrating the obtained liposome, in particular suitable for dehydration by spray-drying or spray-freeze drying.

The flow rate of the solution or suspension may be in the range of about 1 ml/min to about 1000 ml/min. More typically, the liposomes were prepared by the method of the invention applying a flow rate of 10 ml/min to 200 ml/min, and more preferably from about 20 ml/min to about 100 ml/min.

According to another embodiment, the pressure used for extruding the suspension or solution comprising lipids through the porous device and the pressure for passing the suspension or solution through the nozzle may be substantially identical, in particular may be between 0.5 bar and 1200 bar. More typically, the liposomes can be prepared by the method of the invention with 5 bar to 600 bar, preferably from about 10 bar to about 500 bar and more preferably from about 20 bar to about 150 bar.

Pumping the lipid/liposome suspension or solution with the pressure and flow rate employed in the inventive method as described above may be achieved by suitable high pressure pumps that are generally known in the art. When piston pumps are employed, the suspension may be drawn into the pump head itself. External pumping of feed stock from the feed reservoir by an external pumping device is possible. The invention is not limited to piston pumps and any suitable pumping means may be used including diaphragm pumps. In embodiments with a sufficient hydrostatic head and thus a sufficient flow rate and pressure differential the pump is not necessary.

It is another aspect of the current invention that the use of pressures between about 10 bar and about 500 bar enables the preparation of liposomes using a starting suspension or solution comprising a high lipid concentration of up to about 400 mM lipid. By using high pressure for the porous device extrusion, clogging of the extrusion porous device can be prevented even at high lipid concentrations. Prior art liposome sizing techniques employing polycarbonate porous devices used pressures less than 7 bar, and thus were limited to lipid concentrations of 60 µmol/ml. In the current process, rapid extrusion rates on the order of 20 ml/min and above are still achieved for high lipid concentrations when pressures are higher, e.g. when pressures in the range of 20 bar to 150 bar are used.

The current method as described in its embodiments enables the rapid preparation of liposomes in a continuous, reproducible mode by a single extrusion through a porous device and subsequent passing through a nozzle. The increase of the pressure used for performing the method results in a reduced size and a narrower size distribution of the liposomes prepared by the process.

It is another aspect of the current invention, that the liposomes prepared by the inventive method have a high size homogeneity as embodied by a narrow size distribution. The high size homogeneity or narrow size distribution is characterized by a low polydispersity index. Preferably the liposomes prepared by the current invention are characterized by a polydispersity index lower than about 0.3, more preferably lower than about 0.2, most preferably lower than about 0.1. Polydispersity (PI) of the liposomes may be determined by photon correlation spectroscopy (PCS) using a Malvern Zetasizer Nano (Malvern Instruments; UK).

In another aspect of the invention, liposomes may be dehydrated subsequent to their preparation to preserve the liposomes for prolonged storage. Suitable methods for dehydrating liposomes include spray-drying or spray-freeze-drying. Formation of droplets by the atomisation of the liposome comprising suspension represents the initial step in both methods of dehydration. Liposomes my also be dehydrated under reduced pressure. In an especially preferred embodiment, liposomes may be dehydrated by percolative drying as described in the co-owned European patent application "Percolative drying for the preparation of particles" filed on 27.10.2006.

Its has been described above, that it is an object of the present invention, that a suspension or solution comprising lipids, preferably comprising lipid particles, most preferably liposomes is passed through a nozzle, preferably at a high pressure. By this way, the suspension is atomised, leading to a formation of droplets from the suspension or solution.

Therefore, it is another aspect of the present invention, to provide a continuous method for preparation of liposomes from a starting suspension or solution comprising lipids, and dehydrating said liposomes by spray-drying or spray-freeze-drying, whereby the method comprises extruding a suspension or solution comprising lipids through a porous device and subsequently passing the suspension or solution through a nozzle, whereby the suspension is atomised to form droplets.

Drying of the liposomes after droplet formation may be achieved by contacting the droplets with a gas stream, preferably a heated gas stream, to obtain solid particles. Preferably, the used gaseous stream is an inert gas. The drying gas can preferably be a low-oxygen gas containing less than 0.1 vol. %, preferably less than 0.05 vol. %, oxygen or an oxygen-free gas. Inert gaseous are increasing the safety of a heated drying systems that contains highly flammable solutions, by pumping nitrogen, carbon dioxide, helium, neon, argon, krypton, xenon and radon or some other inert gas in order to displace oxygen. The effect of these systems is to either completely remove the oxygen, or reduce it to a negligible level. In a preferred embodiment of the invention nitrogen is used as an inert gaseous. In an other embodiment of the invention the inert gas protects the active ingredients and excipients containing in the formulation. Preferably the spray-drying is performed in a suitable device for spray drying. The spray-drying may for example be performed in a drying tower. The dehydrated liposomes are separated from the gas stream and collected.

The method according to the invention can be performed in a pass-through or loop operation as known as inert-spray-drying device to an a skilled person in the field. In the pass-through or loop operation, inert gas can be heated as the drying gas. The drying gas can then be used to spray dry the dryer feed of the suspension containing the suspension with liposomes. The spray drying can be performed under excess pressure, normal pressure or partial vacuum. A favourable process pressure range can exist if powder conforming to specification is produced with the drying gas at the maximum permissible system temperature and at maximum capacity. Since the introduction of oxygen into the system must be avoided, the plant can preferably be operated under normal pressure or slight overpressure of 0 to 200 mbar above ambient pressure. The heated drying gas inside the process chamber can display a temperature of 10 °C to 500°C such that the solvent evaporates when the dryer feed comes into contact with the drying gas. More typically, the suspension were dried by the method of the invention with 30°C to 250°C, and more preferably from about 80°C to about 200°C.

Separation of the dried liposomal product can take place with discharge of the product. The product stream can be separated from the waste-gas stream by suitable means, for example using filters or cyclones, optionally cooled and if necessary stored under a protective gas atmosphere or packed. If surface filters with pressure surge cleaning are used for dust collection, cleaning can be performed with any oxygen-free gas, but preferably with preheated inert gas or a split stream of the drying gas.

The solvents can be condensed and the exhaust air is recycled by processing through the inert loop. In the pass-through operation fresh drying gas is repeatedly fed into the process chamber and the exhaust gas leaving the process chamber discarded. The method according to the invention also can be performed in a gas recycling operation in the loop operation the used drying gas is free of solvents. The difference from the pass-through operation is that the exhaust gas can be recycled and conditioned by energy input in such a way that it can be used again as the drying gas. During conditioning, the liquid components evaporated in the process chamber can be partially removed from the exhaust gas such that they too can be recycled. As complete as possible a gas recycling with removal of the excess solvents and inert gases is desirable from an economic perspective.

A preferred embodiment of an apparatus for the preparation of dry liposomes according to the invention is shown in Fig. 1.

Freezing of the droplets in a spray-freezing step may be achieved by contacting said droplets with a cryogenic fluid. The cryogenic fluid can be a cold gas or a cryogenic liquid. Cryogenic liquids are chilled liquids like argon, helium, hydrogen, nitrogen, oxygen, methane, carbon dioxide, nitrous oxide, isopentane, hexane, or ethanol and other fluids like hydrocarbon fluids or mixtures thereof. In a preferred embodiment of the invention nitrogen is used as a cryogenic liquid. The contacting of the droplets with the cryogenic fluid results in the formation of frozen particles. Key, variable operating parameters include rotor speed, atomising gas pressure, annular gap gas pressure, air volume, solution spray-rate and spray-gun nozzle diameter, slit width for the fluidised bed and inlet and outlet air temperatures.

It is a feature of the present invention that the liposomes comprised in the particles obtained by the freezing step may be dehydrated under reduced pressure without thawing said particles. Under reduced pressure, water and other solvents are removed from frozen particles by sublimation. The reduction of pressure enables decreased drying times for thermally labile compounds.

In order to obtain solid particles in which liposomes are incorporated or embedded, it is useful to select a weight ratio of the hydrophilic excipient to the colloidal systems which favours an acceptable degree of incorporation of the colloid. For example, if the aqueous liquid comprises more colloidal systems than hydrophilic excipient, some of the colloidal systems may not be incorporated in or embedded in the solid particles produced by the method of the invention. Therefore, it is preferred that the weight ratio of the hydrophilic excipient to the colloidal system is at least about 1, and more preferably at least about 2, such as about 3 to about 5 or even more. On the other hand, an extremely high ratio should be avoided because it might lead to rather large powder volumes for a specific content of colloidal systems and thus to a large volume of liquid composition after reconstitution. Thus, ratios of more than about 50 or even more than 100 are less preferred. According to the invention, ratios of less than about 50 and particularly of less than about 20 are particularly preferred.

As pointed out, the method of the invention also allows the preparation of liposomes in suspension and conversion into dry particles in a single-pass procedure. The dry particles comprise a hydrophilic excipient, which acts as a carrier for the liposomes which are embedded or incorporated in the dry particles. The method can be conducted at relatively low temperatures, so that it is particularly advantageous for the drying and stabilisation of liposomes which are thermally labile or which comprise a thermally labile active agent in concern of spray drying.

Liposomes which have been dehydrated by the inventive method may be rehydrated in a suitable aqueous medium. It has surprisingly been found that suspensions comprising liposomes and/or lipid particles may be prepared and additionally dried by the method of the invention in a possible one step procedure in such a way that they can easily be reconstituted. The mild conditions, in particular lower spray-drying temperatures using aqueous organic solutions in combination with inert gas make the method extremely useful for preparing and drying thermally labile liposomes.

The invention discloses dehydrated liposomes which have been obtained by the disclosed method. These liposomes have an average diameter smaller than about 500 nm, preferably smaller than about 200 nm after rehydration of the liposomes in an aqueous medium, preferably in a medium which is suitable for a pharmaceutical application, most preferably water for injection. The disclosed dehydrated liposomes have a polydispersity index of lower than about 0.5, preferably lower than about 0.2, most preferably lower than about 0.15, after rehydration. The liposomes may be comprised in dry particles with an average size between about 10 µm and about 1000 µm preferably between about 50 µm and about 500 µm.

The liposomes, the dry particles, or a powder comprising the same, as obtained by the method of the invention may be used in the manufacture of a medicinal or a diagnostic preparation. If the particles fulfil all requirements of a pharmaceutical dosage form, it may be used as such, and filled directly into appropriate primary packaging containers. Alternatively, the particles may be further processed. For example, it may be mixed with further active and/or inactive ingredients as for example pharmaceutically acceptable carriers, or it may be compressed into a pharmaceutical tablet.

After the reconstitution of the dry particles containing liposomes, such as with water for injection or other buffer systems, the resulting formulations can be used for parenteral (e.g. i.v. or locoregional) injection, oral administration, pulmonary or nasal inhalation. Solvents, in case they are used in the preparation method, will be extracted during the method of invention below permissible or detectable limits, which is a particular advantage of the invention over methods known in the prior art.

### EXAMPLES

The following examples are meant to further illustrate the invention and some of its preferred embodiments without limiting its scope. Further examples and embodiments will easily be derived from the description, optionally in combination with generally known technical information, in particular in combination with known principles of designing and using colloidal drug carrier systems and spray-drying, inert spray drying or spray-freeze drying.

### Analytics of the liposomes

The particle size (Z-average) and polydispersity (PI) of the liposomes were analyzed by photon correlation spectroscopy (PCS) using a Malvern Zetasizer Nano (Malvern Instruments; UK). The lipid concentration of DOTAP-CI and DOPC was analyzed by HPLC using an UV/VIS detector at 205 nm. Separation and quantification of the components was carried out using a C8 LiChrospher 60 RP-selected B column (250 x 4mm, 5 µm particle size) with a C18 pre-column.

### Liposome preparation

Cationic liposomes with total lipid content between 10 to 400 mM were prepared by ethanol injection in the aqueous organic phase. DOTAP-Cl (1,2-dioleoyl-3-trimethylammonium-propane-chloride) and DOPC (1,2-dioleoyl-sn-glycero-3-phosphocholine), both from Avanti Polar Lipids (Alabaster, USA), were dissolved in ethanol. Multilamellar liposomes formed spontaneously upon the injection into 10.5% trehalose solution (Ferro Pfanstiehl, USA) up to an organic content of 30%. The suspensions were passed through the polycarbonate membranes of 100 or 200 nm pore size and nozzle with an orifice diameter of 0.1 and 0.2 once through in order to obtain liposomes with defined size distribution.

### Analytics of particles

Residual moisture was determined by a coulometric Karl Fischer titrator with a head-space oven (Analytic Jena AG, Germany). Particle size distribution was measured with a He-Ne laser beam equipped laser diffraction analyzer (Mastersizer X, Malvern, Germany).

### Example 1

Cationic liposomes with total lipid content of 10 mM were prepared by ethanol injection as dislosed for example by Mundus et al in WO 2004/002468. DOTAP-CI (1,2-dioleoyl-3-trimethylammonium-propane-chloride) 5 mM and DOPC 5 mM (1,2-dioleoyl-sn-glycero-3-phosphocholine), both from Avanti Polar Lipids (Alabaster, USA), were dissolved in ethanol. Multilamellar liposomes formed spontaneously upon the injection into 10.5% trehalose solution (Ferro Pfanstiehl, USA). The aqueous-organic multilamellar liposome dispersion as prepared by ethanol injection was passed through a porous device connected to a orifice nozzle using an ISCO DM 260 syringe pump. The applied flow rates and resulting pressures are indicated in Table 1. The porous device consisted of a polycarbonate membrane (Osmonics Inc., USA) having a pore size of 200 nm supported from both sides by a polyester drain disk (Osmonics Inc., USA) followed by a stainless steel frit having a filtration porosity of 5µm further supporting the membrane and drain an the membrane/drain disk side facing the connected nozzle. A 121 VG ¼ nozzle (Schlick, Germany) with an orifice diameter of 0.1 mm was used. Liposomes were collected after passing the nozzle and analysed.
The properties of liposomes prepared by the described method with differing lipid concentrations, flow rates and pressures are shown in Table 1.

**Table1**

| Lipid concentration [mM] | Flow rate [ml/min] | Pressure [bar] | Ethanol concentration [w/v] | Average diameter [nm] | Polydispersity index |
|---|---|---|---|---|---|
| 10 | 5 | 3.1 | 2.5 | 153.5 ± 7.7 | 0.23 ± 0.01 |
| 10 | 10 | 6.1 | 2.5 | 150.0 ± 5.5 | 0.23 ± 0.02 |
| 10 | 40 | 45.2 | 2.5 | 144.5 ± 4.9 | 0.19 ± 0.01 |
| 10 | 60 | 138.0 | 2.5 | 130.0 ± 2.8 | 0.19 ± 0.01 |
| 10 | 80 | 338.0 | 2.5 | 134.0 | 0.17 |
| 10 | 100 | 378.5 | 2.5 | 127.5 ± 7.7 | 0.14 ± 0.02 |
| 40 | 20 | 150.0 | 2.5 | 175.5 | 0.24 |
| 80 | 20 | 170.2 | 2.5 | 180.5 | 0.25 |

### Example 2

The method was conducted in accordance with example 1, using a polycarbonate membrane having a pore size of 100 nm passing. The properties of liposomes prepared by the described method with different flow rates and pressures are shown in Table 2.

**Table 2**

| Lipid concentration [mM] | Flow rate [ml/min] | Pressure [bar] | Ethanol concentration [w/v] | Average diameter [nm] | Polydispersity index |
|---|---|---|---|---|---|
| 10 | 5 | 4.6 | 2.5 | 144.5 ± 2.1 | 0.26 ± 0.01 |
| 10 | 10 | 7.7 | 2.5 | 146.5 ± 4.9 | 0.19 ± 0.05 |
| 10 | 40 | 49.6 | 2.5 | 133.5 ± 3.5 | 0.18 ± 0.01 |
| 10 | 60 | 200.1 | 2.5 | 123.0 ± 2.8 | 0.16 ± 0.05 |
| 10 | 80 | 344.9 | 2.5 | 119.5 ± 2.1 | 0.17 ± 0.02 |

### Example 3

The method was conducted in accordance with example 1, using a nozzle having an orifice of 0.2 mm. The properties of liposomes prepared by the described method with differing lipid concentrations, flow rates and pressures are shown in Table 3.

**Table 3**

| Lipid concentration [mM] | Flow rate [ml/min] | Pressure [bar] | Ethanol concentration [w/v] | Average diameter [nm] | Polydispersity index |
|---|---|---|---|---|---|
| 10 | 5 | 2.6 | 2.5 | 162.5 ± 12.0 | 0.25 ± 0.02 |
| 10 | 10 | 5.4 | 2.5 | 152.0 ± 12.7 | 0.24 ± 0.01 |
| 10 | 40 | 20.0 | 2.5 | 137.5 ± 7.7 | 0.18 ± 0.01 |
| 10 | 60 | 62.1 | 2.5 | 124.5 ± 210.6 | 0.17 ± 0.01 |
| 10 | 80 | 65.0 | 2.5 | 119.0 | 0.17 |

### Example 4

The method was conducted in accordance with example 1 expect that the ethanol concentration of the aqueous-organic dispersion was varied between 10% and 30% [w/v]. The properties of liposomes prepared by the described method with differing ethanol concentrations, flow rates and pressures are shown in Table 4.

**Table 4**

| Lipid concentration [mM] | Flow rate [ml/min] | Pressure [bar] | Ethanol concentration [w/v] | Average diameter [nm] | Polydispersity index |
|---|---|---|---|---|---|
| 10 | 5 | 1.0 | 10 | 220 ± 43.1 | 0.47 ± 0.18 |
| 10 | 15 | 8.5 | 10 | 168 ± 61.5 | 0.46 ± 0.06 |
| 10 | 60 | 396.0 | 10 | 80 | 0.29 |
| 10 | 5 | 0.8 | 20 | 144 ± 9.8 | 0.46 ± 0.05 |
| 10 | 15 | 6.0 | 20 | 98 ± 3.5 | 0.35 ± 0.06 |
| 10 | 30 | 23.2 | 20 | 70 ± 2.1 | 0.29 ± 0.01 |
| 10 | 60 | 296.0 | 20 | 59 | 0.30 |
| 10 | 5 | 1.5 | 30 | 114 ± 23.3 | 0.28 ± 0.01 |
| 10 | 15 | 7.5 | 30 | 75 ± 24.7 | 0.13 ± 0.01 |
| 10 | 30 | 22.1 | 30 | 50 ± 6.3 | 0.16 ± 0.05 |
| 10 | 60 | 115.0 | 30 | 43 | 0.09 |

### Example 5

The method was conducted in accordance to example 4 expect that a polycarbonate membrane having a pore size of 200 nm was employed. The properties of liposomes prepared by the described method with differing ethanol concentrations, flow rates and pressures are shown in Table 5.

**Table 5**

| Lipid concentration [mM] | Flow rate [ml/min] | Pressure [bar] | Ethanol concentration [w/v] | Average diameter [nm] | Polydispersity index |
|---|---|---|---|---|---|
| 10 | 5 | 1.7 | 10 | 118 ± 2.8 | 0.24 ± 0.03 |
| 10 | 30 | 13.5 | 10 | 106 ± 2.1 | 0.21 ± 0.02 |
| 10 | 60 | 440.0 | 10 | 82 | 0.20 |
| 10 | 5 | 2.5 | 20 | 84 ± 3.5 | 0.21 ± 0.05 |
| 10 | 15 | 14.5 | 20 | 84 ± 0.7 | 0.16 ± 0.02 |
| 10 | 30 | 59.7 | 20 | 71 ± 3.5 | 0.16 ± 0.05 |
| 10 | 60 | 309.2 | 20 | 60 | 0.28 |
| 10 | 5 | 1.7 | 30 | 71 ± 0.7 | 0.12 ± 0.02 |
| 10 | 15 | 8.1 | 30 | 69 ± 4.9 | 0.10 ± 0.04 |
| 10 | 30 | 30.2 | 30 | 52 ± 2.8 | 0.09 ± 0.00 |
| 10 | 60 | 118 | 30 | 42 | 0.07 |

### Example 6

The method was conducted in analogy to example 1 except that the aqueous-organic dispersion was pumped through the polycarbonate membrane having a pore size of 200 nm passing the nozzle having an orifice of 0.1 mm or 0.2 mm diameter the nozzle at a 20 ml/min flow rate with a ethanol concentration of 10 or 30 % [w/v]. The resulting atomisation pressure was between 20 and 22 bar.

After atomisation the dispersion into droplets by passing the dispersion through the nozzle (4), the moisture was evaporated by contacting the droplets with the inert drying gas in a drying tower (5) (B-290 and B-295, Buchi, Switzerland). The resulting dry particles were separated from the inert gas by a cyclone (Buchi, Switzerland). The drying inert gas temperature was heated to an inlet temperature of 200°C using the heater (feature of Buchi B290), resulting in an outlet temperature of about 75°C. The inert drying gas, gaseous nitrogen was supplied with a constant drying volumetric flow rate of 38 m³/hour. The separated solid and smooth particles had a particle size in the range of 50 to 500 µm with a residual moisture below 3 %. After reconstitution with water for injection a liposomal dispersion was obtained.

The resulting average diameter and polydispersity index of the liposomes is shown in Table 6. The set-up of the equipment is depicted in Figure1.

**Table 6**

| Nozzle Diameter [mm] | Inlet Temp. [°C] | Outlet Temp. [°C] | Lipid conc. [mM] | Flow rate [ml/ min] | Pressure [bar] | Ethanol conc. [w/v] | Average diameter [nm] | Polydispersity index |
|---|---|---|---|---|---|---|---|---|
| 0.1 | 200 | 75 | 10 | 20 | 22 | 10 | 138 | 0.14 |
| 0.1 | 200 | 78 | 10 | 20 | 20 | 30 | 162 | 0.42 |
| 0.2 | 200 | 87 | 10 | 20 | 8 | 10 | 144 | 0.17 |
| 0.2 | 200 | 87 | 10 | 20 | 7 | 20 | 179 | 0.33 |

### Figure Legend

**Figure 1:** Apparatus for the preparation and dehydration of liposome in a single device.
(1) Syringe pump
(2) Porous device
(3) Inlet temperature
(4) Nozzle
(5) Drying tower
(6) Outlet temperature
(7) Cyclone
(8) Product
(9) Inert loop (optionally)

**Figure 2:** Apparatus for the preparation of liposomes in a single-pass mode.
(10) Stock solution
(11) pump
(12) filter holder
(13) drain disc
(14) membrane, preferably polycarbonate membrane
(15) porous material, preferably a frit
(16) nozzle

## Claims

1. A method for the preparation of liposomes in a single-pass mode, comprising the steps:
(a) providing a suspension or solution comprising lipids,
(b) extruding the suspension or solution of step (a) through a porous device, and subsequently
(c) passing said suspension or solution of step (b) through a nozzle, and
(d) optionally collecting the liposomes formed after step (c).

2. A method according to claim 1, wherein step (c) comprises atomising the suspension or solution into droplets comprising liposomes.

3. A method according to claim 1 or 2, further comprising the steps
(d) dehydrating the liposomes of step (c) and
(e) optionally collecting the dehydrated liposomes formed after (d), and
(f) optionally rehydrating the dehydrated liposomes of step (d) in an aqueous medium.

4. A method according to claim 3, wherein the dehydration step (d) is performed by spray-drying or by spray-freeze drying.

5. A method according to any one of claims 1-4, wherein the suspension or solution of step (a), preferably comprises lipid particles.

6. A method according to any one of claims 1-5, wherein the lipid particles of the suspension or solution of step (a) are MLVs.

7. A method according to any one of claims 1-6, wherein the particle size distribution of the liposomes obtained after step (c) is reduced compared to the particle size distribution of the lipid particles of the suspension of step (a).

8. A method according to any one of claims 1-7, wherein the liposomes obtained after step (c) are substantially homogeneous.

9. A method according to any one of claims 1-8, wherein the liposomes obtained after step (c) have a polydispersity index of lower than 0.3, preferably lower than 0.1.

10. A method according to any one of claims 1-9, wherein the liposomes obtained after step (c) have an average diameter of between about 50 nm and about 500 nm, preferably between about 50 nm and 200 nm.

11. A method according to any one of claims 1-10, wherein the liposomes obtained after step (c) are unilamellar liposomes.

12. A method according to any one of claims 1-11, wherein the suspension or solution of step (a) has a lipid concentration of up to 400 nM.

13. A method according to any one of claims 1-12, wherein the suspension or solution of step (a) comprises at least one cationic lipid, preferably DOTAP.

14. A method according to any one of claims 1-13, wherein the suspension or solution of step (a) and/or the liposomes obtained after step (c) comprise at least one active compound.

15. A method according to claim 14, wherein the active compound is a pharmaceutical active compound, e.g. an antimicrobial, antifungal, antiviral, cytostatic or cytotoxic agent.

16. A method according to any one of claims 1-15, wherein the suspension or solution of step (a) comprises an aqueous medium.

17. A method according to claim 16, wherein the aqueous medium comprises at least one further liquid constituent at least partially miscible with water, preferably an organic solvent.

18. A method according to claim 17, wherein the organic solvent is an alcohol or a ketone such as methanol, ethanol, propanol, butanol, acetone, methylethylketone, DMF, DMSO or a mixture thereof, preferably ethanol.

19. A method according to claims 17 or 18, wherein the ratio between water and the further liquid constituent is between about 70:30 and about 40:60 (v/v), most preferably between about 80:20 and about 60:40 (v/v).

20. A method according to any one of claims 1-18, wherein the suspension or solution of step (a) comprises a hydrophilic excipient.

21. A method according to any one of claims 1-21, wherein in step (b) the pressure of the suspension or solution is about the same on both sides of the porous device.

22. A method according to any one of claims 1-21, wherein the porous device of step (b) has a pore size of between about 50 and about 300 nm, preferably of between about 100 and about 200 nm.

23. A method according to any one of claims 1-22, wherein the porous device is a membrane, preferably a polycarbonate membrane.

24. A method according to any one of claims 1-23, wherein the porous device, preferably the membrane is supported by at least one drain disk from at least one side.

25. A method according to claim 24, wherein the at least one drain disk is supported by at least one frit.

26. A method according to any one of claims 1-25, wherein the nozzle of step (c) is an orifice nozzle.

27. A method according to any one of claims 1-26, wherein the nozzle of step (c) has a diameter between about 0.05 mm and about 1 mm, preferably between about 0.1 mm and about 0.2 mm.

28. A method according to any one of claims 1-27, wherein the suspension or solution is extruded through the porous device in step (b) and is subsequently passed through the nozzle in step (c) with a flow rate between about 1 ml/min and 1000 ml/min, preferably about 20 ml/min and 100 ml/min.

29. A method according to any one of claims 1-28, wherein the suspension or solution is extruded through the porous device in step (b) and is subsequently passed through the nozzle in step (c) with a pressure between about 0.5 bar and 1200 bar, preferably about 5 bar and 600 bar.

30. A method according to any one of claims 3-29, wherein the dehydration step (d) is performed with a stream of gas, preferably heated gas.

31. A method according to claim 30, wherein the gas is an inert gas, preferably nitrogen.

32. A method according to any one of claims 3-31, wherein the dehydration step (d) is performed by spray-drying, preferably in a pass-through or loop operation.

33. A method according to any one of claims 3-29, wherein the dehydration step (d) is performed by a spray-freeze drying, whereby freezing is achieved by contacting the liposomes obtained in step (c) with cryogenic fluid.

34. A method according to claim 33, wherein the cryogenic fluid is a cold gas or a cryogenic liquid, preferably nitrogen.

35. A method according to any one of claims 33-34, wherein frozen particles resulting from said spray freezing are subsequently dehydrated, preferably under reduced pressure.

36. Liposomes prepared by a method of claims 1-35.

37. Deydrated liposomes prepared by a method of any one of claims 3-36, comprising an average diameter smaller than about 500 nm, preferably smaller than about 200 nm after rehydration in an aqueous medium.

38. Dehydrated liposomes according to claim 37, comprising a polydispersity index of about lower than 0.2, preferably of about lower than 0.15 after rehydration in an aqueous medium.

39. Dehydrated liposomes according to any of claims 37-38, which are comprised in dry particles with an average size between about 10 µm and about 1000 µm, preferably between about 50 µm and about 500 µm.

40. The use of liposomes according to any one of claims 36-39 for the manufacture of a medicament.

41. An apparatus for the preparation of liposomes comprising a porous device which is connected to a nozzle.

42. An apparatus according to claim 41 comprising
(i) a container for supplying a solution or suspension comprising lipids,
(ii) a porous device,
(iii) a nozzle,
(iv) means for connecting the container (i) with the porous device (ii) by a conduit,
(v) means for generating pressure, and
(vi) optionally means for dehydrating the solution or suspension comprising liposomes and
(vii) optionally means for collecting the obtained liposomes.

43. An apparatus according to claims 41 or 42, wherein the nozzle is is an orifice nozzle.

44. An apparatus according to any one of claims 41-43, wherein the nozzle has a diameter between about 0.05 mm and about 1 mm, preferably between about 0.1 mm and about 0.2 mm.

45. An apparatus according to any of claims 41-44, wherein the nozzle is comprised in the means for dehydration.

46. An apparatus according to any of claims 41-45, wherein the porous device has a pore size of between 50 nm and about 300 nm, preferably between about 100 nm and about 200 nm.

47. An apparatus according to claims 41-46, wherein the porous device is a membrane, preferably a polycarbonate membrane.

48. An apparatus according to claims 41-47, wherein said membrane is supported by at least one drain disk from at least one side.

49. An apparatus according to claim 48, wherein the at least one drain disk is supported by at least one frit.
